(19) Europäisches Patentamt
European Patent Office
Office européen des brevets

(11) Publication number : 0 577 446 A2

(12) EUROPEAN PATENT APPLICATION

(21) Application number : 93401344.2

(22) Date of filing : 26.05.93

(51) Int. Cl.[5] : **C12P 41/00**, C12P 7/04, C12P 7/22, C12P 7/62

(30) Priority : 28.05.92 JP 162140/92

(43) Date of publication of application : 05.01.94 Bulletin 94/01

(84) Designated Contracting States : DE FR GB

(71) Applicant : **Showa Shell Sekiyu Kabushiki Kaisha**
**2-5, Kasumigaseki 3-chome Chiyoda-ku**
**Tokyo (JP)**

(72) Inventor : **Miyazawa, Toshifumi**
**2-12-2, Kitaochiai, Suma-ku**
**Kobe-shi, Hyogo 654-01 (JP)**
Inventor : **Hirose, Katutoshi, c/o KOBE NATURAL PRODUCT CHEMICAL CO., LTD., 10-6, Kamishinchi 3-chome**
**Nishi-ku, Kobe-shi, Hyogo 674 (JP)**
Inventor : **Takagi, Yoshihiro, c/o KOBE NATURAL PRODUCT CHEMICAL CO., LTD., 10-6, Kamishinchi 3-chome**
**Nishi-ku, Kobe-shi, Hyogo 674 (JP)**
Inventor : **Otomatsu, Toshihiko, c/o KOBE NATURAL PRODUCT CHEMICAL CO., LTD., 10-6, Kamishinchi 3-chome**
**Nishi-ku, Kobe-shi, Hyogo 674 (JP)**
Inventor : **Kurita, Sota, c/o KOBE NATURAL PRODUCT CHEMICAL CO., LTD., 10-6, Kamishinchi 3-chome**
**Nishi-ku, Kobe-shi, Hyogo 674 (JP)**
Inventor : **Suzuki, Yoshiichi, c/o SHOWA SHELL SEKIYU K.K.**
**2-5, Kasumigaseki 3-chome, Chiyoda-ku 3-chome**
**Tokyo 100 (JP)**
Inventor : **Nishikawa, Koujiro**
**10-3, Tamondai 2-chome, Tarumi-ku**
**Kobe-shi, Hyogo 655 (JP)**

(74) Representative : **Bourgognon, Jean-Marie et al**
**Cabinet Flechner 22, Avenue de Friedland**
**F-75008 Paris (FR)**

(54) **Process for preparing optically active halogen-containing alcohols.**

(57) Optically active, halogen-containing alcohols useful as intermediate of liquid crystal compounds are prepared in which an enzyme originated from microorganisms selected from the genera of Chromobacterium, Alcaligenes, Candida, Geotrichum, Humicola, Mucor, Penicillium, Rhizopus and Pseudomonas or an enzyme originated from wheat malt, such as wheat germ, is allowed to react, in organic solvents, with a racemic, halogen-containing alcohol of the formula [I] :

$$R_1 - \underset{}{\overset{OH}{\overset{|}{CH}}} - R_2 \quad ..... \quad [I]$$

wherein $R_1$ is a halogen-containing alkyl group having 1 to 4 carbon atoms ; and $R_2$ is a group selected from alkyl and aralkyl groups having 4 to 16 carbon atoms, substituted and unsubstituted aryl groups and $-CH_2-COO-R_5$ wherein $R_5$ is an alkyl or aralkyl group having 2 to 16 carbon atoms, and a vinyl ester of the formula [II] :

$$R_3 - \overset{O}{\overset{\|}{CO}} - R_4 \quad ..... \quad [II]$$

wherein $R_3$ is an alkyl, substituted alkyl or alkenyl group having 1 to 11 carbon atoms ; and $R_4$ is a substituted or unsubstituted vinyl group having 2 to 4 carbon atoms, until the corresponding (S)-type or (R)-type isomer of the compound [I] is obtained.

EP 0 577 446 A2

The present invention relates to a process for preparing a halogen-containing alcohol of high optical purity by subjecting a vinyl ester and a racemic, halogen-ontaining alcohol to an asymmetric esterification reaction using an esterase. The optically active, halogen-containing alcohols of the invention are useful as intermediates for liquid crystal materials, pharmaceuticals, pesticides and others.

Methods for preparing optically active, halogen-containing alcohols involve asymmetric syntheses through an organic synthetic reaction and optical resolutions using a diastereomer. However, these methods have much difficulties in obtaining products of high optical purity and in synthesizing them in a large amount, so that established practical methods are few. Optical resolution methods have also been reported by th present inventors in Japanese Patent [KOKAI] Publications Nos. 282340/1990, 233243/1989 and 233244/1989. These methods pertain to enzymatic reactions in an aqueous solution, which have certain problems from technical points of view, for example, inferior dispersibility of substrates, and difficulties in obtaining a high optical yield, in synthesizing in a large scale, and in separating and recovering enzymes.

To overcome these technical problems, assymetric enzymatic reaction in an organic solvent has been focused recently. This method has also difficulties in not being able to attain high optical purity because of a partial reverse reaction due to reversibility of esterification reaction.

More recently, there has been reported that high optical purity is attained by an irreversible esterification reaction using a vinyl ester derivative (J. Org. Chem., 1988, 53, 3127-3219). However, nothing was reported on halogen-containing alcohols, except that an optical resolution through ester-exchanging reaction of a trifluoromethyl group-containing alcohol with a vinyl ester using Lipase P originated from Pseudomonas sp. was tried. In this case, however, the reaction hardly proceeds and no optical resolution is attained (J. Chem. Soc., Chem. Commun., 1988, 1459-1461, particularly Table 1).

An object of the present invention is to provide a novel process for efficiently preparing an optically active, halogen-containing alcohol from the corresponding racemic, halogen-containing alcohol in organic solvents.

The first aspect of the present invention relates to a process for preparing an optically active or enantiomeric halogen-containing alcohol represented by the formula [III]:

$$R_1 - \overset{\overset{\displaystyle OH}{|}}{\underset{*}{CH}} - R_2 \qquad \cdots\cdots\cdots \quad [III]$$

wherein $R_1$ is a halogen-containing alkyl group having 1 to 4 carbon atoms; $R_2$ is a group selected from alkyl and aralkyl groups having 4 to 16 carbon atoms, substituted and unsubstituted aryl groups and $-CH_2-COO-R_5$ wherein $R_5$ is an alkyl or aralkyl group having 2 to 16 carbon atoms; and * means an asymmetric carbon atom, which is characterized by subjecting a racemic, halogen-containing alcohol represented by the formula [I]:

$$R_1 - \overset{\overset{\displaystyle OH}{|}}{CH} - R_2 \qquad \cdots\cdots\cdots \quad [I]$$

wherein $R_1$ and $R_2$ have the same meanings as above, and a vinyl ester represented by the formula [II]:

$$R_3 - \overset{\overset{\displaystyle O}{\|}}{CO} - R_4 \qquad \cdots\cdots\cdots \quad [II]$$

wherein $R_3$ is an alkyl, substituted alkyl or alkenyl group having 1 to 11 carbon atoms; and $R_4$ is a substituted or unsubstituted vinyl group having 2 to 4 carbon atoms, to an asymmetric esterification reaction in organic solvents through action of an enzyme originated from microorganisms selected from the group consisting of genera of

Chromobacterium
Alcaligenes
Candida
Geotrichum
Humicola
Mucor

Penicillium
Rhizopus
Pseudomonas sp.

or an enzyme originated from wheat malt, such as wheat germ.

The secnd aspect of the present invention relates to a process for preparing an optically active or enantiomeric halogen-containing alcohol represented by the formula [III]:

$$R_1-\underset{*}{\overset{\overset{OH}{|}}{CH}}-R_2 \qquad \cdots\cdots\cdots \quad [III]$$

wherein $R_1$ is a halogen-containing alkyl group having 1 to 4 carbon atoms; $R_2$ is a group selected from alkyl and aralkyl groups having 4 to 16 carbon atoms, substituted and unsubstituted aryl groups and $-CH_2-COO-R_5$ wherein $R_5$ is an alkyl or aralkyl group having 2 to 16 carbon atoms; and * means an asymmetric carbon atom, which is characterized by subjecting a racemic, halogen-containing alcohol represented by the formula [I]:

$$R_1-\overset{\overset{OH}{|}}{CH}-R_2 \qquad \cdots\cdots\cdots \quad [I]$$

wherein $R_1$ and $R_2$ have the same meanings as above, and a vinyl ester represented by the formula [II];

$$R_3-\overset{\overset{O}{\|}}{CO}-R_4 \qquad \cdots\cdots\cdots \quad [II]$$

wherein $R_3$ is an alkyl, substituted alkyl or alkenyl group having 1 to 11 carbon atoms; and $R_4$ is a substituted or unsubstituted vinyl group having 2 to 4 carbon atoms, to an asymmetric ester-exchanging reaction in organic solvents through action of an enzyme originated from microorganisms selected from the group consisting of genera of

Chromobacterium
Alcaligenes
Candida
Geotrichum
Humicola
Mucor
Penicillium
Rhizopus
Pseudomonas sp.

or an enzyme originated from wheat malt, such as wheat germ, to obtain an optically active, halogen-containing ester represented by the formula [IV]:

$$R_3-\overset{\overset{O}{\|}}{CO}-\underset{*}{\overset{\overset{R_1}{|}}{CH}}-R_2 \quad \cdots\cdots\cdots \quad [IV]$$

wherein $R_1$, $R_2$ and $R_3$ have the same meanings as above and * means an asymmetric carbon atom, and separating and then hydrolyzing said ester.

The present invention is summarized according to the following reaction formulas (1), (2) and (3):

$$(\text{R-type})/(\text{S-type})\text{ROH} + R_3COO - \overset{\displaystyle CH_3}{\overset{|}{C}} = CH_2 \xrightarrow{\text{enzyme}}$$

$$R_3COOR(\text{S-type}) + CH_2 = \overset{\displaystyle OH}{\overset{|}{CH}} - CH_3 + (\text{R-type})\text{ROH} \quad \ldots (1)$$

$$CH_2 = \overset{\displaystyle OH}{\overset{|}{CH}} - CH_3 \xrightarrow[\text{to ketone}]{\text{tautomerism}} CH_3 - \overset{\displaystyle O}{\overset{||}{C}} - CH_3 \quad \ldots (2)$$

$$R_3COOR(\text{S-type}) \xrightarrow{\text{hydrolysis}} (\text{S-type})\text{ROH} \quad \ldots (3)$$

(wherein R represents an

$$R_1 - \overset{|}{CH} - R_2$$

group.)

In the above reaction formulas, the product, ROH, in the formula (1) is shown as (R)-type, and the product, ROH, in the formula (3) is shown as (S)-type. However, which the products in the formulas (1) and (3) are (R)-type or (S)-type, depends upon an enzyme employed. For the convenience of explanation, herein, the product in the formula (1) is referred to (R)-type, and that in the formula (3), (S)-type.

If the ester component in the reaction does not have a vinyl group, the reaction in the formula (1) essentially proceeds reversibly, so that (S)-type $R_3$ COOR once formed causes again a reverse esterification reaction, by which optical purity is lowered. If the ester component contains a vinyl group, the vinyl alcohol component formed in the formula (1) causes tautomerism to a ketone or aldehyde as shown in the formula (2), thus, no reversible reaction of the formula (1) occurs. Accordingly, decrease in optical purity of the formed (R)-type ROH is prevented.

$R_1$ in the formula [III] includes $CH_2F$, $CHF_2$, $CF_3$, $CClF_2$, $CCl_2F$, $CF_3CCl_2$, $C_2F_5$ and $C_3F_7$.

$R_2$ in the formula [III] includes straight chain or branched alkyl groups, such as n-butyl, isobutyl, n-pentyl, 2-methylbutyl and n-hexyl groups; phenyl and substituted phenyl groups, such as tolyl and xylyl groups; and benzyl and phenethyl groups.

$R_3$ includes alkyl groups, such as methyl, ethyl, n-propyl, n-butyl, isobutyl, n-hexyl, n-octyl and n-decyl groups; substituted alkyl groups, such as monochloromethyl, monobromomethyl, monochloroethyl and monochloropropyl groups; and alkenyl groups, such as $CH_2=CH-$, $CH_3CH=CH-$, $CH_2=C(CH_3)-$, $CH_3CH=CHCH=CH-$ and $CH_2=CH-(CH_2)_9-$.

$R_4$ includes vinyl, isopropenyl and n-butenyl groups.

$R_5$ includes ethyl, propyl, n-butyl, isobutyl, n-hexyl, n-octyl, n-decyl, n-hexadecyl and benzyl groups.

Compounds represented by the formula [I] include the following ones:

$$HO - \overset{\displaystyle CH_2F}{\overset{|}{CH}} - C_6H_{13}$$

$$CHF_2$$
$$HO - CH - C_6H_{13}$$

$$CHF_3$$
$$HO - CH - C_6H_{13}$$

$$CCl_2F$$
$$HO - CH - C_6H_{13}$$

$$C_2F_5$$
$$HO - CH - C_6H_{13}$$

$$C_3F_7$$
$$HO - CH - C_6H_{13}$$

$$CH_2F$$
$$HO - CH - C_8H_{17}$$

$$CHF_2$$
$$HO - CH - C_8H_{17}$$

$$CF_3$$
$$HO - CH - C_8H_{17}$$

$$CCl_2F$$
$$HO - CH - C_8H_{17}$$

$$C_2F_5$$
$$HO - CH - C_8H_{17}$$

$C_3F_7$
$HO - CH - C_8H_{17}$

$CF_3$
$HO - CH - C_4H_9$

$CF_3$
$HO - CH - C_5H_{11}$

$CF_3$
$HO - CH - C_7H_{15}$

$CF_3$
$HO - CH - C_9H_{19}$

$CF_3$
$HO - CH - C_{10}H_{21}$

$CF_3$
$HO - CH - C_{16}H_{33}$

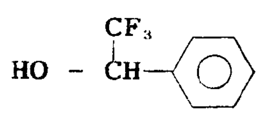

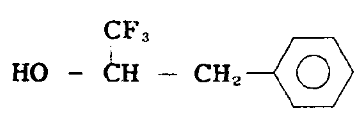

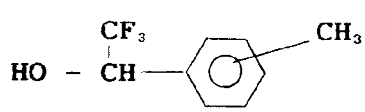

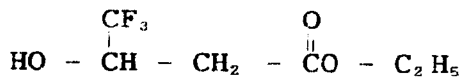

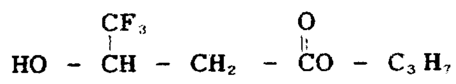

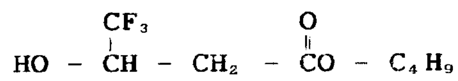

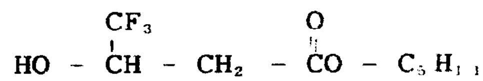

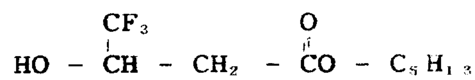

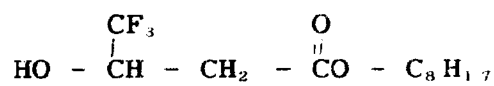

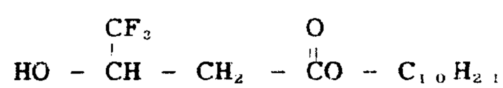

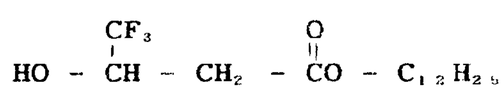

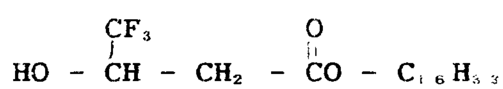

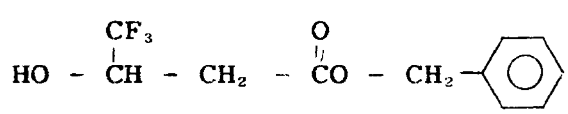

Compounds represented by the formula [II] include the following ones:

$$CH_3 - \overset{O}{\overset{\|}{C}}O - CH = CH_2$$

$$CH_3 - CH_2 - \overset{O}{\overset{\|}{C}}O - CH = CH_2$$

$$CH_3 - (CH_2)_2 - \overset{O}{\overset{\|}{C}}O - CH = CH_2$$

$$CH_3 - \overset{CH_3}{\overset{|}{C}}H - \overset{O}{\overset{\|}{C}}O - CH = CH_2$$

$$CH_3 - (CH_2)_3 - \overset{O}{\overset{\|}{C}}O - CH = CH_2$$

$$CH_3 - (CH_2)_4 - \overset{O}{\overset{\|}{C}}O - CH = CH_2$$

$$CH_3 - (CH_2)_5 - \overset{O}{\overset{\|}{C}}O - CH = CH_2$$

$$CH_3 - \overset{O}{\overset{\|}{C}}O - \overset{CH_3}{\overset{|}{C}} = CH_2$$

$$CH_3 - \overset{O}{\overset{\|}{C}}O - CH = CH - C_2H_5$$

$$HO - \underset{*}{\overset{CH_2F}{\overset{|}{C}H}} - C_6H_{13}$$

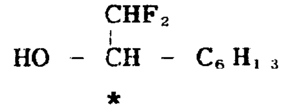

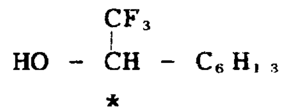

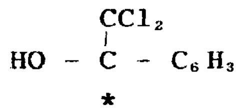

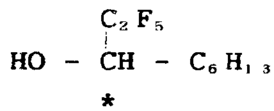

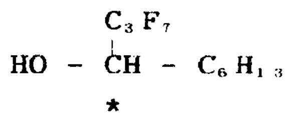

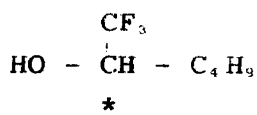

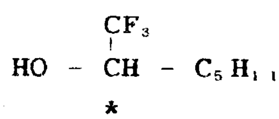

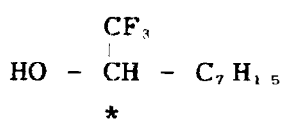

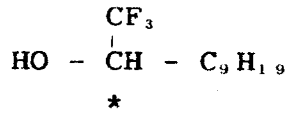

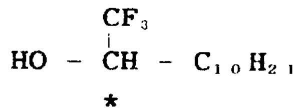

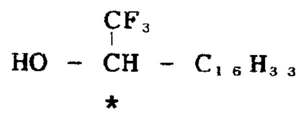

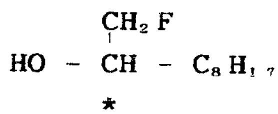

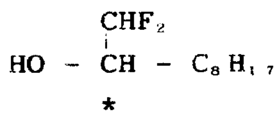

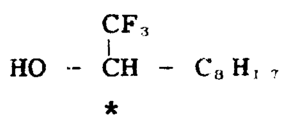

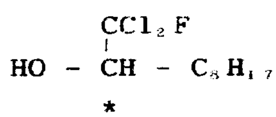

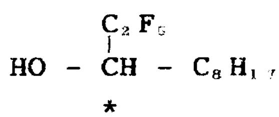

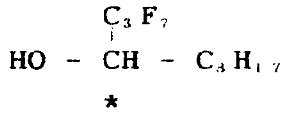

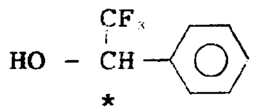

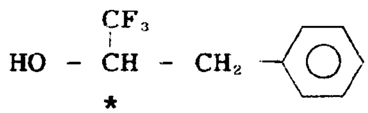

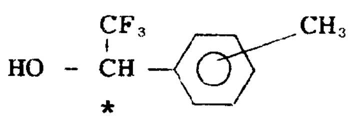

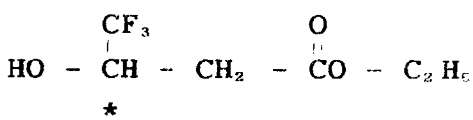

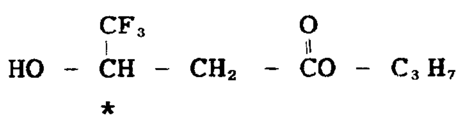

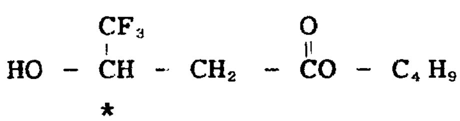

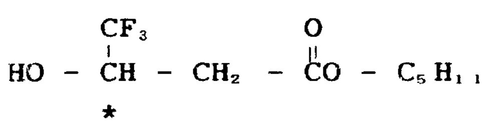

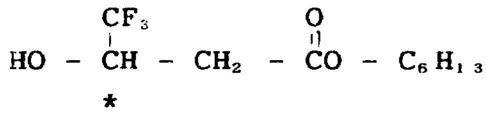

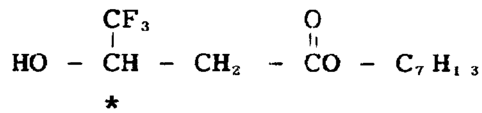

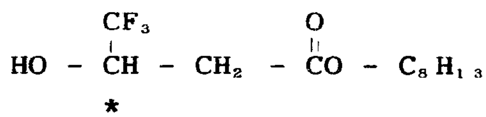

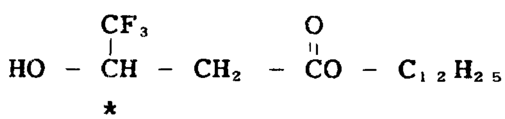

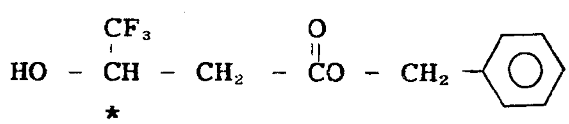

Organic solvents employed in the invention include aliphatic hydrocarbons having 5 to 10 carbon atoms, for example, n-hexane and n-heptane; alicyclic hydrocarbons having 6 to 9 carbon atoms, for example, cyclohexane; aromatic hydrocarbons having 7 to 9 carbon atoms, for example, toluene; ether solvents, for example, diethyl ether, diisopropyl ether and tetrahydrofuran; and chlorinated aliphatic hydrocarbons having 1 or 2 carbon atoms, for example, 1,2-dichloroethane and chloroform, among which the chlorinated solvents are preferred.

Enzymes in the present invention, which are to be used for optical resolution through an asymmetric ester-exchanging reaction in an organic solvent, should be those which accelerate the asymmetric ester-exchanging reaction without being deactivated in organic solvents.

As a result of extensive studies, the present inventors have found that enzymes mentioned in Table 1 exhibit efficient optical resolution performance for the asymmetric ester-exchanging reaction of vinyl esters and halogen-containing alcohols in organic solvents, thereby to obtain the halogen-containing alcohols of high optical purity.

Namely, enzymes employed in the process of the invention include those originated from microorganisms selected from the group consisting of genera of

Chromobacterium
Alcaligenes
Candida
Geotrichum
Humicola
Mucor
Penicillium
Rhizopus
Pseudomonas sp.

and those originated from wheat malt, such as wheat germ. Enzymes other than those illustrated above may be used so far as they are active for the asymmetric ester-exchanging reaction in organic solvents. Particularly preferable are those mentioned in Table 1.

Table 1

| Origins | Tradenames | Manufactures |
|---|---|---|
| Chromobacterium viscosum | Lipase LP | Asahi Kasei |
| Alcaligenes sp. | Alcaligenes sp. Lipase | Cosmo Bio |
| Candida cylindracea | Lipase Type VII | SIGMA |
| Candida cylindracea | Lipase OF-360 | Meito Sangyo |
| Candida lipolytica | Lipase from Candida lipolytica | Fluka |
| Geotrichum candidum | Geotrickum candidum Lipase | Cosmo Bio |
| Humicola lanuginosa | Humicola lanuginosa Lipase | Cosmo Bio |
| Mucor miehei | Mucor Lipase M | Cosmo Bio |
| Penicillium cyclopium | Penicillim Lipase C | Cosmo Bio |
| Penicillium roqueforti | Lipase from Penicillium roqueforti | Fluka |
| Rhizopus arrhizus | Lipase Type XI | SIGUMA |
| Rhizopus delemar | Lipase from Rhizopus delemar | Fluka |
| Rhizopus niveus | Lipase from Rhizopus niveus | Fluka |
| Wheat germ | Lipase Type I | SIGMA |
| Pseudomonas sp. | Lipase SAM-II | Fluka |
| Pseudomonas Sp. | Lipase Type XIII | SIGMA |

Amount of the enzyme used is preferably not less than 20% by weight, more preferably 35 to 65% by weight, per the reaction substrate. Even an amount as small as 5% by weight accelerates the reaction, but such a case needs a longer period of time, for example, 300 to 600 hours.

Reaction temperature may normally be a temperature at which the enzyme is not deactivated, namely, a temperature within the range of 20 to 70°C, preferably, 50 to 60°C. The optimum temperature varies depending upon each enzyme employed.

The reaction period of time is not limitative, but generally within the range of 1 to 300 hours.

Optimum asymmetric ester-exchanging ratio to obtain a high optical purity in the present invention varies depending upon the combination of enzymes and substrates. In case of a combination of pentafluoro-2-alkanol and Lipase LP, for example, a high optical purity of not less than 90% is obtained if the reaction is ceased at an exchanging ratio of not less than 50%, preferably 55 to 60% or more. A high ester exchanging ratio is possible, but it tends to decrease yield.

The ester-exchanging ratio may be measured by assaying directly the reaction mixture using a gas chromatography

Conditions for gas chromatography

Column: SE-30
Initial temperature: 250°C
Column temperature: 130°C
Detector: FID
Injecting 3 $\mu\ell$ of the reaction mixture.

When a resoluted optically active, halogen-containing alcohol is obtained according to the present reaction,

its enantiomer remains in the reaction system in the form of an ester. Thus, the enantiomer which is an optically active antipode is obtained by separating and recovering the ester and subjecting it to hydrolysis with an alkali.

Procedures for determining optical purity in the present invention are as follows.

About 5 mg of a sample alcohol is weighted, and dissolved in 0.5 ml of dry toluene. A mixture of the solution with about 5 mg of 3,5-dinitrophenyl isocyanate is subjected to pulverization and then mixed with about 5 to 10 mg of 4-dimethylaminopyridine. The mixture is stirred for 1 hour at 60 to 70°C, and then concentrated at 60 to 70°C. The residue is dissolved in about 15 ml of ethyl ether, washed sequentially with 1 M HCl twice, $H_2O$, 1 M $NaHCO_3$ and saturated NaCl solution, and dried over anhydrous sodium sulfate. The ether layer in about 8 μl volume is assayed using high pressure liquid chromatography (HPLC).

<u>Conditions for HPLC</u>

| | |
|---|---|
| Column: | SUMICHIRAL OA-4000 |
| Solvent: | n-Hexane: $CHCl_3$: IPA = 50 : 30 : 1 |
| Flowing amount: | 1 ml/min. |
| Detection wave length: | 254 nm |

The invention will more fully be explained with respect to examples, which are, however, never construed to limit the invention.

Part 1: Examples for optical resolution reaction of 1,1,1,2,2-pentafluoro-3-undecanyl alcohol:

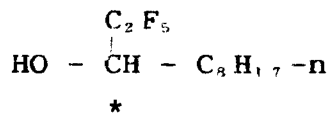

Example 1

To 30 ml of n-hexane were added 2.4 g of 1,1,1,2,2-pentafluoro-3-undecanyl alcohol, 3 g of vinyl butyrate and 750 mg of Lipase LP originated from <u>Chromobacterium viscosum</u>, made by Asahi Kasei. The mixture was well stirred to keep a dispersion for 72 hours at 30°C to proceed with a reaction. Process of the reaction was pursued by checking ester-exchanging ratio every 8 hours using a gas chromatography. When the exchanging ratio reached 65%, the reaction was ceased by removing the lipase through a suction filtration. The filtrate was concentrated and then subjected to a silica gel column chromatography wherein n-hexane/isopropyl ether = 5/1 by volume was used, for separation and purification. The first fraction obtained was an ester, and the second fraction was 0.61 g of the titled optically active 1,1,1,2,2-pentafluoro-3-undecanyl alcohol.

NMR ($CDCl_3$ δ TMS inner standard)

0.84 (3H t J = 6.7 Hz)
1.1 - 1.43 (12H m)
1.45 - 1.62 (2H m)
1.63 - 1.75 (1H m)
3.87 - 4.20 (1H m)

HPLC analysis in the form of 3,5-dinitrophenyl isocyanate derivative showed that it was the (R) isomer with an optical purity of 86% e.e.

Example 2

Procedures in Example 1 were repeated except that 3 g of vinyl acetate was used in place of the vinyl butyrate. After 48 hour reaction at an exchanging ratio of 50%, the resulting optically active alcohol had an optically purity of 39% e.e.

Example 3

Procedures in Example 1 were repeated except that 3 g of vinyl propionate was used in place of the vinyl butyrate. After 20 hour reaction at an exchanging ratio of 64%, the resulting optically active alcohol had an optical purity of 93.5% e.e.

Example 4

Procedures in Example 2 were repeated except that the same amount of diisopropyl ether was used as the solvent in place of the n-hexane. After 168 hour reaction at an exchanging ratio of 54%, the resulting optically active alcohol had an optical purity of 65% e.e.

Example 5

Procedures in Example 1 were repeated except that the 3 g of vinyl propionate as the ester and 30 ml of 1,2-dichloroethane as the solvent were used in place of the vinyl butyrate and n-hexane, respectively, at a reaction temperature 50°C. After 90 hour reaction at an exchanging ratio of 58%, the resulting optically active alcohol had an optical purity of 97.6% e.e.

Example 6

Procedures in Example 5 were repeated except that 30 ml of tetrahydrofuran (THF) was used as the solvent in place of the 1,2-dichloroethane. After 383 hour reaction at an exchanging ratio of 59%, the resulting optically active alcohol had an optical purity of 90% e.e.

Example 7

Procedures in Example 5 were repeated except that the same amount of n-hexane was used as the solvent in place of the 1,2-dichloroethane. After 116 hour reaction at an exchanging ratio of 62%, the resulting optically active alcohol had an optical purity of 72% e.e.

Example 8

Procedures in Example 7 were repeated except that 750 mg of Lipase Type XIII originated from Pseudomonas sp., made by SIGMA, was used in place of the Lipase LP. After 330 hour reaction at an exchanging ratio of 64%, the resulting optically active alcohol had an optical purity of 9.6% e.e.

Example 9

Procedures in Example 5 were repeated except that 750 mg of Lipase SAM-II originated from Pseudomonas sp., made by Fluka, was used in place of the Lipase LP. After 330 hour reaction at an exchanging ratio of 46%, the resulting optically active alcohol had an optical purity of 28.8% e.e.

Example 10

Procedures in Example 5 were repeated except that 750 mg of Lipase Type VII originated from Candida cylindracea, made by SIGMA, was used in place of the Lipase LP. After 330 hour reaction at an exchanging ratio of 6%, the resulting optically active alcohol had an optical purity of 5.1% e.e.

Part 2: Examples for optical resolution reaction of 1,1,1-trifluoro-2-decanol:

$$HO - \underset{*}{\overset{\overset{CF_3}{|}}{CH}} - C_8H_{17}$$

Example 11

To 30 ml of chloroform were added 2.4 g of 1,1,1-trifluoro-2-decanol, 3 g of vinyl propionate and 750 mg of Lipase LP originated from Chromobacterium viscosum, made by Asahi Kasei. The mixture was well stirred to keep a dispersion for 70 hours at 5°C to proceed with a reaction. Process of the reaction was pursued by checking ester-exchanging ratio every 8 hours using a gas chromatography. The reaction was ceased at an exchanging ratio of 76% by removing the lipase through a suction filtration. The filtrate was concentrated and subjected to a silica gel chromatography wherein n-hexane/isopropyl ether = 5/1 by volume was used, for separation and purification. The first fraction obtained was an ester, and the second fraction was 0.45 g of the titled optically active 1,1,1-trifluoro-2-decanol alcohol.

NMR ($CDCl_3$ δ TMS inner standard)

0.86 (3H t J = 7.0 Hz)
1.20 - 1.40 (11H m)
1.45 - 1.60 (2H m)
1.60 - 1.70 (1H m)
3.70 - 3.85 (2H m)

HPLC analysis in the form of 3,5-dinitrophenyl isocyanate derivative showed that it was the (R) isomer with an optical purity of 95% e.e.

Example 12

Procedures in Example 11 were repeated except that 30 ml of tetrahydrofuran (THF) was used as the solvent in place of the chloroform. After 95 hour reaction at an exchanging ratio of 75% , the resulting optically active alcohol had an optical purity of 82% e.e.

Example 13

Procedures in Example 11 were repeated except that 30 ml of toluene was used as the solvent in place of the chloroform. After 95 hour reaction at an exchanging ratio of 78%, the resulting optically active alcohol had an optical purity of 81% e.e.

Example 14

Procedures in Example 11 were repeated except that 30 ml of hexane was used as the solvent in place of the chloroform. After 70 hour reaction at an exchanging ratio of 72%, the resulting optically active alcohol had an optical purity of 60% e.e.

Part 3: Examples for optical resolution reaction of 1,1,1,2,2,3,3-heptafluoro-4-decanol:

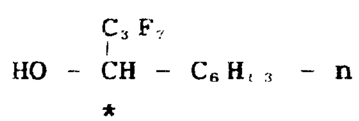

Example 15

To 20 ml of 1,2-dichloroethane were added 870 mg of 1,1,1,2,2,3,3-heptafluoro-4-decanol, 1 g of vinyl propionate and 290 mg of Lipase LP originated from Chromobacterium viscosum, made by Asahi Kasei. The mixture was well stirred to keep a dispersion at 50°C for 72 hours to proceed with a reaction. Process of the reaction was pursued by checking ester-exchanging ratio every 8 hours using a gas chromatography. When the exchanging ratio reached 63%, the reaction was ceased by removing the lipase through a suction filtration. The filtrate was concentrated and subjected to a silica gel chromatography wherein n-hexane/isopropyl ether = 5/1 by volume was used, for separation and purification. The first fraction obtained was an ester, and the second fraction was 160 mg of the titled optically active 1,1,1,2,2,3,3-heptafluoro-4-decanol alcohol.

HPLC analysis in the form of 3,5-dinitrophenyl isocyanate derivative showed that it was the (R) isomer with an optical purity of 46.5% e.e.

Example 16

Procedures in Example 15 were repeated except that 860 mg of vinyl acetate in place of the vinyl propionate, and 550 mg of the same Lipase LP were used. After 53 hour reaction at an exchanging ratio of 63%, the resulting optically active alcohol had an optical purity of 34.1% e.e.

Example 17

Procedures in Example 15 were repeated except that 1 g of Lipase SMA-II originated from Pseudomonas sp., made by Fluka, was used, in place of the Lipase LP. After 95 hour reaction at an exchanging ratio of 6.9%, the resulting optically active alcohol had an optical purity of 2.3% e.e.

Part 4: Examples for optical resolution reaction of 1,1,1,2,2,3,3-heptafluoro-4-dodecanol:

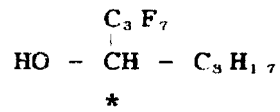

Example 18

Procedures in Example 15 were repeated except that 950 mg of 1,1,1,2,2,3,3-heptafluoro-4-dodecanol was used in place of the 1,1,1,2,2,3,3-heptafluoro-4-decanol. After 75 hour reaction at an exchanging ratio of 64.5%, the resulting optically active alcohol had an optical purity of 46.8% ee.

Part 5: Examples for optical resolution reaction of ethyl 4,4,4-trifluoro-3-hydroxybutyrate:

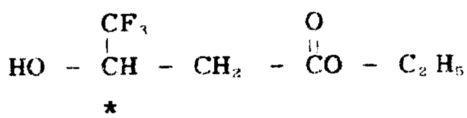

Example 19

To 20 ml of 1,2-dichloroethane were added 530 mg of ethyl 4,4,4-trifluoro-3-hydroxybutyrate, 1 g of vinyl propionate and 290 mg of Lipase LP originated from Chromobacterium viscosum, made by Asahi Kasei. The mixture was well stirred to keep a dispersion at 50°C for 11 hours to proceed with a reaction. The reaction was ceased at an exchanging ratio of 52.9% by removing the lipase through a suction filtration. The filtrate was concentrated and subjected to a silica gel chromatography wherein n-hexane/isopropyl ether = 5/1 by volume was used, for separation and purification to obtain 130 mg of optically active ethyl 4,4,4-trifluoro-3-hydroxybutyrate.

HPLC analysis in the form of 3,5-dinitrophenyl isocyanate derivative showed that its optical purity was 1.8% e.e.

Example 20

Procedures in Example 19 were repeated except that Lipase SAM-II originated from Pseudomonas sp., made by Fluka, was used in place of the Lipase LP. After 169 hour reaction at an exchanging ratio of 61.9%, the resulting optically active ethyl 4,4,4-trifluoro-2-hydroxybutyrate had an optical purity of 74% e.e.

Example 21

Procedures in Exammple 19 were repeated except that 750 mg of Lipase OF-360 made by MEITO SANGYO was used, in place of the Lipase LP. After 170 hour reaction at an exchanging ratio of 5.3%, the resulting optically active ethyl 4,4,4-trifluoro-3-hydroxybutyrate had an optical purity of 0.5% e.e.

Example 22

To 30 ml of THF-water (1 : 1) was dissolved 1.63 g of 1,1,1-trifluoro-2-decyl propionate, which had been obtained as the first fraction in silica gel column chromatography in Example 11, and the solution was mixed with 0.27 g of sodium hydroxide. The mixture was stirred for 18 hours at 30°C. After addition of 100 ml of water, the reaction mixture was extracted with 50 ml of diethyl ether, and the ether layer was dried over anhydrous

sodium sulfate. After removal of the solvent, 1.02 g of optically active 1,1,1-trifluoro-2-decanol was obtained.

HPLC analysis in the form of 3,5-dinitrophenyl isocyanate derivative showed it was (S) isomer with an optical purity of 31% e.e.

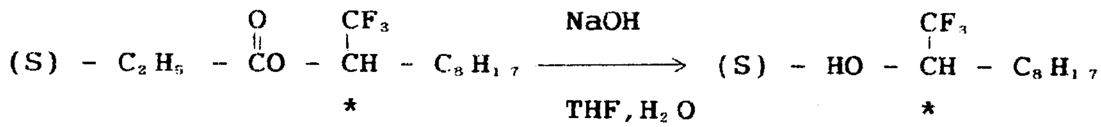


## Claims

1. A process for preparing an optically active, halogen-containing alcohol represented by the formula [III]:

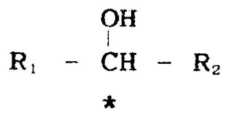 ..... [III]

wherein $R_1$ is a halogen-containing alkyl group having 1 to 4 carbon atoms; $R_2$ is a group selected from alkyl and aralkyl groups having 4 to 16 carbon atoms, substituted and unsubstituted aryl groups and $-CH_2-COO-R_5$ wherein $R_5$ is an alkyl or aralkyl group having 2 to 16 carbon atoms; and * means an asymmetric carbon atom, which comprises subjecting a racemic, halogen-containing alcohol represented by the formula [I]:

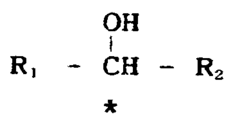 ..... [I]

wherein $R_1$ and $R_2$ have the same meanings as above, and a vinyl ester represented by the formula [II]:

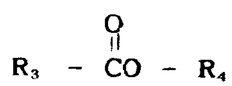 ..... [II]

wherein $R_3$ is an alkyl, substituted alkyl or alkenyl group having 1 to 11 carbon atoms; and $R_4$ is a substituted or unsubstituted vinyl group having 2 to 4 carbon atoms, to an asymmetric esterification reaction in organic solvents through action of an enzyme originated from microorganisms selected from the group consisting of genera of

<u>Chromobacterium</u>
<u>Alcaligenes</u>
<u>Candida</u>
<u>Geotrichum</u>
<u>Humicola</u>
<u>Mucor</u>
<u>Penicillium</u>
<u>Rhizopus</u>
<u>Pseudomonas sp</u>.

or an enzyme originated from wheat malt.

2. A process for preparing an optically active, halogen-containing alcohol represented by the formula [III]:

$$R_1 - \underset{*}{\overset{\overset{OH}{|}}{CH}} - R_2 \qquad ..... \ [III]$$

wherein $R_1$ is a halogen-containing alkyl group having 1 to 4 carbon atoms; $R_2$ is a group selected from alkyl and aralkyl groups having 4 to 16 carbon atoms, substituted and unsubstituted aryl groups and $-CH_2-COO-R_5$ wherein $R_5$ is an alkyl or aralkyl group having 2 to 16 carbon atoms; and * means an asymmetric carbon atom, which comprises subjecting a racemic, halogen-containing alcohol represented by the formula [I]:

$$R_1 - \underset{*}{\overset{\overset{OH}{|}}{CH}} - R_2 \qquad ..... \ [I]$$

wherein $R_1$ and $R_2$ have the same meanings as above, and a vinyl ester represented by the formula [II]:

$$R_3 - \overset{\overset{O}{\|}}{CO} - R_4 \qquad ..... \ [II]$$

wherein $R_3$ is an alkyl, substituted alkyl or alkenyl group having 1 to 11 carbon atoms: and $R_4$ is a substituted or unsubstituted vinyl group having 2 to 4 carbon atoms, to an asymmetric ester-exchanging reaction in organic solvents through action of an enzyme originated from microorganisms selected from the group consisting of genera of

Chromobacterium
Alcaligenes
Candida
Geotrichum
Humicola
Mucor
Penicillium
Rhizopus
Pseudomonas sp.

or an enzyme originated from wheat malt, to obtain an optically active, halogen-containing ester represented by the formula [IV]:

$$R_3 - \overset{\overset{O}{\|}}{CO} - \underset{*}{\overset{\overset{R_1}{|}}{CH}} - R_2 \qquad ..... \ [IV]$$

wherein $R_1$, $R_2$ and $R_3$ have the same meanings as above and * means an asymmetric carbon atom, and separating and then hydrolyzing said ester.

**3.** A process for preparing optically active, halogen-containing alcohols according to Claim 1 or 2 wherein the organic solvents are aliphatic hydrocarbon solvents, alicyclic hydrocarbon solvents, aromatic hydrocarbon solvents, ether solvents, or chlorinated solvents.